(19) Europäisches Patentamt
European Patent Office
Office européen des brevets

(11) EP 0 536 070 B1

(12) **FASCICULE DE BREVET EUROPEEN**

(45) Date de publication et mention de la délivrance du brevet:
**27.05.1998 Bulletin 1998/22**

(51) Int. Cl.[6]: **C07C 209/36**, C07C 209/38, C07C 211/52, B01J 27/22

(21) Numéro de dépôt: **92420349.0**

(22) Date de dépôt: **02.10.1992**

# (54) Matériau à base de carbure(s) de tungstène, catalyseur et procédé utile pour l'hydrogénation de dérivé nitro ou nitroso aromatique utilisant ce catalyseur

Material aus Wolframcarbid, Katalysator und Verfahren zur Hydrierung von nitro- oder nitrosoaromatischen Derivaten durch Verwendung dieses Katalysators

Tungsten carbide based material, catalyst and useful process for the hydrogenation of nitro or nitroso aromatic derivatives using this catalyst

(84) Etats contractants désignés:
**AT BE CH DE DK ES FR GB GR IE IT LI LU NL PT SE**

(30) Priorité: **04.10.1991 FR 9112269**
**04.10.1991 FR 9112270**
**10.07.1992 FR 9208581**

(43) Date de publication de la demande:
**07.04.1993 Bulletin 1993/14**

(73) Titulaire: **RHODIA CHIMIE**
**92408 Courbevoie Cédex (FR)**

(72) Inventeurs:
- **Jacquot, Roland**
  **F-69110 Saint-Foy les Lyon (FR)**
- **Mercier, Claude**
  **F-69005 Lyon (FR)**

(74) Mandataire:
**Ricalens, François et al**
**RHODIA CHIMIE**
**Direction de la Propriéte Industrielle**
**25, quai Paul Doumer**
**92408 Courbevoie Cédex (FR)**

(56) Documents cités:
- **JOURNAL OF THE CHEMICAL SOCIETY, PERKIN TRANSACTIONS 2. vol. 8, 1975, LETCHWORTH GB pages 827 - 829 GY RGY HORANYI ET AL. 'kinetics of the liquid-phase hydrogenation of aromatic nitro-compounds in the presence of tungsten carbide catalyst'**
- **JOURNAL OF THE CHEMICAL SOCIETY, PERKIN TRANSACTIONS 2. vol. 10, 1973, LETCHWORTH GB pages 1400 - 1402 GY RGY VERTES ET AL. 'selective catalytic behaviour of tungsten carbide in the liquid-phase hydrogenation of organic compounds'**
- **CHEMICAL ABSTRACTS, vol. 78, no. 25, 25 Juin 1973, Columbus, Ohio, US; abstract no. 159108y, HORANYI, G ET AL. 'tungsten carbide as catalyst for liquid (aqueous) phase heterogeneous catalytic hydrogenation' page 375 ;colonne 1 ;**
- **CHEMICAL ABSTRACTS, vol. 81, no. 7, 19 Août 1974, Columbus, Ohio, US; abstract no. 37392c, page 373 ;colonne 1 ;**
- **CHEMICAL ABSTRACTS, vol. 81, no. 8, 26 Août 1974, Columbus, Ohio, US; abstract no. 41902m, VERTES, G ET AL. 'application of tungsten carbide and tungsten oxide in liquid-phase catalytic hydrogenation.' page 302 ;colonne 2 ;**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 536 070 B1

## Description

La présente invention a pour objet un procédé et un catalyseur d'hydrogénation de composés nitrés ou nitroso (nitrosés) aromatiques. Elle concerne, plus particulièrement la préparation d'amines halogénées à partir de composés nitrés et halogénés aromatiques et notamment l'accès aux amines - para substituées par un groupe X=OH, Cl, Br, F, alcoxyle et acyloxyle par transposition de Bamberger sur l'hydroxylamine intermédiaire.

Lors de la mise en oeuvre d'un procédé d'hydrogénation, sur un dérivé aromatique nitré portant des atomes d'halogène liés au noyau aromatique, il se produit consécutivement, à la transformation du groupe nitro en groupe aminés et indissociablement, un phénomène d'hydrogénolyse de la liaison carbone halogène pour donner, d'une part le noyau déshalogéné et d'autre part des acides halohydriques. Ce phénomène est connu depuis très longtemps puisqu'il a été décrit en 1904 par P. Sabatier et A. Mailhe.

De nombreuses études ont été menées pour éviter cette réaction secondaire tout en permettant de conserver une activité correcte au catalyseur. Ces études ont conduit à diverses solutions qui peuvent être réparties en deux groupes : celles utilisant comme catalyseur d'hydrogénation le nickel de Raney et celles utilisant le platine ou le palladium.

Tous ces brevets décrivent l'utilisation d'un catalyseur modifié.

Dans le premier groupe de techniques, celui mettant en oeuvre les métaux de la mine du platine on peut citer les procédés où l'on utilise comme catalyseur d'hydrogénation, le platine déposé sur charbon éventuellement inhibé par la présence d'adjuvant désigné par le néologisme "d'agent sélectivant", tel que les thioéthers et les disulfures. Bien que le taux de déshalogénation soit très faible, cette technique présente encore de nombreux inconvénients parmi lesquels il convient de citer la formation de sous produits hautement toxiques tel que les dérivés diazotés et le coût très élevé du catalyseur.

Dans le second groupe de techniques, lequel met en oeuvre les métaux de la première ligne du groupe VIII et notamment du nickel sous forme de nickel de Raney, on évite certes la fabrication de dérivés diazoïques, mais la formation d'autres produits parasites reste très élevée, et notamment les produits issus de la hydrogénodéshalogénation (hydrogènolyse de liaisons carbone halogène) ; ce phénomène est particulièrement gênant, dans le cas où l'on désire fabriquer des dérivés fluorés ; il n'est de nos jours guère possible de séparer les anilines fluorées d'avec celles, qui ont un hydrogène à la place du fluor, et ce, à des prix qui soient en rapport avec le prix de vente des dites anilines fluorées.

C'est pourquoi on a cherché à rendre plus sélective la réaction d'hydrogénation, en utilisant des poisons sélectifs des catalyseurs, poisons qualifiés dans ce contexte d'agents sélectivants.

Ainsi, on a préconisé l'utilisation de nickel de Raney, auquel est adjoint un hydroxyde de calcium ou de magnésium. Afin d'éviter la déshalogénation, les températures réactionnelles doivent être très modérées, ce qui ne permet pas d'utiliser ces procédés à l'échelle industrielle.

On a également proposé l'utilisation du nickel de Raney associé à la présence de thiocyanate, d'alcoylamine, d'alcanolamine ou d'une base hétérocyclique, de trialcoylphosphite, de cyanamide, ou de dicyandiamide. Aucune de ces améliorations n'a nettement résolu le problème, d'autant qu'il est plus aisé de mettre en oeuvre comme catalyseur, un matériau présentant intrinsèquement les qualités requises plutôt qu'un catalyseur qu'il convient d'empoisonner avec suffisamment de doigté, pour qu'il ne catalyse que certaines réactions, et ce, sans pour cela perdre la plus grande partie de son efficacité catalytique pour ces réactions.

"Mutatis mutandis", on retrouve ces difficultés lors de la synthèse d'anilides, notamment de composés aminés acylés à partir des dérivés nitrés (ou à partir des intermédiaire identifiés ou non entre les nitrés et les anilines "lato sensu") correspondants

En effet, lors des synthèses classiques d'anilides, il est souvent nécessaire de réaliser la réduction et l'amidation en plusieurs étapes et il est nécessaire d'utiliser des réactifs puissants tels que les anhydrides mixte ou non

C'est pourquoi un, des buts de la présente invention est de fournir un nouveau procédé d'hydrogénation des dérivés aromatiques nitrés et halogénés, notamment, pour la synthèse des anilines halogénées.

Les dérivés halogénés et plus particulièrement fluorés sont très sensibles à l'hydrolyse pendant l'hydrogénation, laquelle dégage deux molécules d'eau par fonction nitro.

C'est pourquoi, un autre but de la présente invention est de fournir un nouveau procédé d'hydrogénation des dérivés aromatiques nitrés et halogénés, notamment, pour la synthèse des anilines halogénées, qui évite l'hydrogénolyse des liaisons carbone halogène.

C'est pourquoi, un autre but de la présente invention est de fournir un nouveau procédé d'hydrogénation des dérivés aromatiques azotés, en général nitrés, notamment pour la synthèse des anilides, qui permettent une amidation quasi simultanée à la formation de la fonction aniline (par aniline, on entend toute amine dont la fonction amine est directement liée par l'azote à un noyau aromatique) en présence de l'acide dont on désire l'anilide en question.

Ces buts et d'autres qui apparaîtront, par la suite, sont atteints au moyen d'un procédé utile pour l'hydrogénation, surtout en phase liquide, de dérivés notamment nitroaromatiques, où ledit dérivé est hydrogéné, en présence de carbure de tungstène activé, la pression partielle d'hydrogène étant au moins égale à 5 atmosphères ($5.10^5$ Pascals).

Il y a plusieurs lustres, voire plusieurs décennies, le carbure de tungstène avait suscité quelqu'espoir dans le

domaine de la catalyse d'hydrogénation, car son spectre d'utilisation semblait différent de celui des autres catalyseurs et notamment de ceux à base de métaux du groupe VIII, mais les résultats n'ont pas été à la hauteur des espérances qu'il avait suscité.

En effet, surtout en phase liquide, la réaction est lente et paresseuse et ne convient pas à une application industrielle ; les différentes mesures préconisées pour pallier cet inconvénient, telles que l'utilisation de surfaces spécifiques élevées, dopage par un metal de la mine du platine n'avaient pas donné les résultats escomptés, tout en limitant les avantages du carbure de tungstène à savoir un spectre d'activité différent de celui des métaux de la mine du platine.

*Ces études proviennent pour l'essentiel d'une équipe hongroise comportant notamment MM. György VERTES, György HORANYI et selon les cas György HOCSNYI, Sandor SZAKACS, Ivan TALCS, Gyula FAZLAT et Pal HORVATH qui ont fait paraître diverses publications.*

*Parmi ces publications il convient de citer la plus ancienne, à savoir la demande de brevet MAGYAR déposée le 28 septembre 1972 sous le Numéro (MA-2410) et publiée le 27 avril 1974 sous le N° 8005 (Chemical Abstracts, vol. 81, n°7, 19 août 1974, Columbus, Ohio, US ; abstract n°37392c, page 373 ; colonne 1). Cette demande décrit un procédé d'hydrogénation à pression atmosphérique, procédé qui utilise du carbure de tungstène en présence d'hydrogène ; selon ce document lorsque l'on utilise des températures proches de ou supérieures à celle du point d'ébullition du solvant utilisé il faut alors accepter une pression totale supérieure à l'atmosphérique, faute de quoi il n'y a plus assez d'hydrogène dans le mélange réactionnel. La cinétique reste faible (dans l'exemple 1 du titre MAGYAR il faut 1g de catalyseur pour hydrogéner 0,2 g de substrat en 10 heures).*

*A la suite de cette demande la même équipe a étudié la cinétique des réactions catalysées par le WC et notamment le rôle des solvants à des pressions au plus égales à la pression atmosphérique (JOURNAL OF THE CHEMICAL SOCIETY, PERKIN TRANSACTIONS 2 VOL.10, 1973 page 827, 829). Dans un dernier article, cette équipe conclut qu'un des meilleurs catalyseurs d'hydrogénation possible à des pressions au plus égales à la pression atmosphérique est un métal de la mine du platine sur un support de carbure de tungstène (JOURNAL OF THE CHEMICAL SOCIETY, PERKIN TRANSACTIONS 2 VOL.8, 1975 page 1400.- 1402).*

A la suite de ces insuccès, les propriétés catalytiques du carbure de tungstène ne furent plus guère considérées que comme des curiosités de laboratoire.

Du fait de cette faible activité catalytique en phase liquide, les études n'avait guère porté que sur des réactions utilisées en pétrochimie pour modifier les hydrocarbures, notamment sur des réactions de déshydrogénation ou de synthèse d'hydrocarbures à partir de gaz à l'eau.

De manière complètement surprenante, l'étude qui a été menée à l'encontre de préjugés sur l'inutilisabilité du carbure de tungstène, a conduit à la conclusion qu'il était possible d'obtenir des cinétiques d'hydrogénation fort acceptables, à condition de travailler à des pressions partielles suffisantes en hydrogène.

En effet, à partir d'un seuil de pression partielle en hydrogène la cinétique augmente brutalement. L'activation à ces pressions intervient très rapidement, et le temps de latence est à peine décelable. Le phénomène est général et a été vérifié pour de nombreuses qualités de carbure de tungstène et de nombreuses réactions.

Selon la sémantique adoptée dans la présente demande, on considère alors que le carbure de tungstène est activé.

Ce seuil est toujours situé au dessus d'une valeur comprise entre 1,5 et 2 atmosphères ; bien évidemment ce seuil dépend des conditions opératoires (dans la présente description on considère que l'atmosphère est interchangeable avec l'unité du système métrique $10^5$ Pascals)

Lorsque l'on travaille à des températures supérieures ou égales à 150°C le seuil est bien situé aux alentours de 2 atmosphères.

Lorsque l'on travaille dans un milieu réactionnel liquide acide (homogène ou hétérogène dont la teneur en acide est au moins égale à 0,1 N de préférence à 1 N) il n'est pas nécessaire d'élever la pression beaucoup au dessus de 2 atmosphères pour atteindre le seuil et ce même à température relativement basse (50 °C environ).

Dans les autre cas l'existence d'un seuil est toujours attestée mais sa valeur est plus difficile à déterminer.

Il est préférable que l'activation ait lieu soit à des pressions supérieures à 10 de préférence à 20 atmosphères soit à une température température supérieure à 100°c de préférence à 150°C soit à une température au moins égale à 100°C et à une pression supérieure à 10 atmosphères.

Il est certes possible de travailler ultérieurement à une pression et/ou une température différente de celle de l'activation. Il est toutefois préférable de de ce placer dans des conditions où l'activation se fait.

La réaction peut, en outre, être conduite dans une large gamme de pressions et de températures, et selon de nombreux modes de réalisation.

Elle peut, ainsi, être réalisée par lot, en semi-continu, en continu, en réacteur agité, ou en lit fixe ruisselant, voire en phase vapeur.

Dans tous les cas, il y a possibilité de récupération et réutilisation du catalyseur, ce qui ajoute à l'intérêt du procédé.

Compte-tenu du fait que l'on travaille dans les conditions d'une catalyse hétérogène, la récupération ultérieure du catalyseur est très facile puisque pouvant être effectuée par des moyens simples, comme une filtration ou une décantation.

La quantité de catalyseur à utiliser n'est pas critique et peut varier dans de larges limites ; généralement on utilise de 0,01 % à 50 % en poids de catalyseur, par rapport à la quantité de substrat engagée.

Selon une des mises en oeuvre préférées de l'invention, la réaction est conduite en milieu solvant.

Elle peut également être conduite sans solvant, les réactifs jouant alors le rôle de solvant.

Un des avantages liés à l'utilisation du carbure de tungstène réside dans la remarquable inertie chimique de ce matériau, notamment vis-à-vis des réactifs protiques et notamment des acides protiques forts. Cette caractéristique est particulièrement intéressante pour les réactions dites de Bamberger. A titre indicatif, on peut rappeler quelques caractéristiques de cette réaction que l'on peut exemplifier de la façon suivante en se plaçant dans le cas du nitrobenzène ou nitrosobenzène

$$\text{H-Ph-NO}_m + m\ H_2 + \text{Nu-H} \xrightarrow{H^+} \text{p ou o Nu-Ph-NH}_2 + m\ H_2O$$

où Nu-H est une molécule protique nucléophile (directement ou par l'intermédiaire de son anion) ; Nu-H peut être un acide ou un composé protique, tel qu'un alcool ou un phénol cela peut être l'eau. La réaction se fait essentiellement en para de la fonction nitro ou nitroso, sauf lorsque cette position est prise par un substituant dont la nucléophilie est sensiblement supérieure à celle de Nu-H. Pour les autres noyaux aromatiques Nu se greffe sur les positions équivalentes à la position ortho et plus généralement à la position para. Pour plus de détails sur la réaction de Bamberger, on peut se reporter à l'abondante littérature sur ce sujet.

La réaction se fait d'autant mieux que le milieu réactionnel, et notamment le solvant, est plus acide. On peut utiliser des acides de Bronstedt, voire de Lewis.

A ce jour, les catalyseurs utilisés étaient choisis parmi ceux à base de métaux de la mine du platine et donc chers, susceptibles de produire de nombreux sous-produits toxiques tels que, des composés azoïques, et présentant une trop forte sensibilité au soufre, ce qui est particulièrement gênant dans le cas des dérivés nitrés, lesquels sont en général obtenus par action de l'acide nitrique en milieu sulfurique et sont stabilisés par adjonction de composés soufrés.

Comme exemples paradigmatiques des fonctions acides utilisables pour les réductions avec "transposition de Bamberger" on peut citer : la première fonction acide des acides phosphoriques et de l'acide sulfurique, ainsi que celles des acides halohydriques et leurs mélanges, quoi qu'ils soient généralement utilisés purs.

Outre leur caractère d'enseignement par l'exemple, les acides ci-dessus sont en général les plus couramment usités pour les transpositions de Bamberger.

Les solvants utilisables pour la réaction de Bamberger sont les solvants usuels en la matière. On rappellera, notamment, que l'on peut utiliser des solvants organiques, protiques ou aprotiques, qui peuvent être constitués au moins partiellement de Nu-H , contenant au moins un acide organique ou minéral.

En général, la réaction est réalisée en l'absence de solvant ou dans un solvant choisi parmi les alcools, les dérivés aromatiques et leurs mélanges ; avantageusement le solvant est choisi parmi les solvants protiques et de préférence parmi l'eau et les alcools, avantageusement le méthanol, et les mélanges en contenant.

Il en va de même avec la réaction d'hydrogénation sans transposition ; seul diffère la présence d'acide fort.

Les températures mises en oeuvre pour conduire la réaction peuvent varier dans de très larges limites.

On peut, ainsi, opérer de la température ambiante jusqu'à, théoriquement, la température d'ébullition du solvant utilisé, en veillant toutefois à ne pas excéder des températures où le substrat et/ou le produit à obtenir pourraient se décomposer ; dans la pratique, on travaille généralement à des températures comprises entre l'ambiante et 400°C, avantageusement entre 20 et 250°C et de préférence entre 50 et 150°C.

En phase liquide, dans la pratique, on travaille généralement à des températures comprises entre l'ambiante et la température d'ébullition du mélange réactionnel, dans les conditions opératoires, avantageusement entre 20°C et 200°C, et de préférence entre 50 et 150°C.

La réaction peut être conduite, de préférence, sous pression autogène dans un réacteur fermé, du type autoclave, contenant une atmosphère d'hydrogène. Dans ce dernier cas, la pression partielle en hydrogène peut aller de 5 à 100 bar (dans la présente description le bar est réputé synonyme de $10^5$ Pascals), et de préférence de 5 à 20 bar.

La réaction est, de préférence, conduite sous agitation, et ceci généralement jusqu'à disparition complète ou quasi-complète du substrat.

En fin de réaction, le catalyseur est séparé du milieu réactionnel par tout moyen de séparation physique connu en soi, tel que par exemple filtration, décantation, élutriation, ou centrifugation.

Les catalyseurs et/ou les solvants ainsi récupérés, après éventuellement purification, peuvent alors être recyclés en tête du procédé.

Un autre but de la présente invention, est de fournir un nouveau catalyseur à base de carbure de tungstène, dont

les caractéristiques cinétiques soient suffisamment améliorées par rapport au carbure de tungstène.

Ce but est atteint par un matériau caractérisé, par le fait, qu'il peut être obtenu en soumettant du carbure de tungstène (ou un composite en contenant) à un traitement d'hydrogénation avantageusement en phase liquide, à une température au moins égale à 50°C avantageusement à 100°C à une pression au moins égale à environ 2 atmosphères avantageusement à environ 5 atmosphères, de préférence à environ 10 atmosphères.

Les valeurs supérieures de pression ne sont pas critiques et ne répondent qu'à des contraintes d'ordre pratique. On peut, à titre purement indicatif, donner, comme limites supérieures pratiques, 100 atmosphères pour la pression ;

Comme valeur supérieure de la température on peut donner une valeur arrondie de 300°C, de préférence de 250°C environ.

Ce matériau peut, notamment, être utilisé comme catalyseur d'hydrogénation, pour la réduction des dérivés nitroaromatiques, ou nitrosoaromatiques. Lorsque les substitutions s'y prêtent, elles permettent les diverses réactions de Bamberger, et ce, d'autant plus facilement que le catalyseur selon l'invention résiste très bien à des conditions acides.

Le catalyseur peut se présenter sous la forme d'un substrat monolithique (nid d'abeilles ou autres), en carbure de tungstène, ou d'un substrat monolithique, revêtu d'une couche en carbure de tungstène, ou bien encore se présenter sous la forme de produits divisés en, ou revêtus de, carbure de tungstène. Par forme divisée, on entend des produits pulvérulents (poudres) et également, les articles obtenus par mise en forme de ces produits (billes, pastilles, boulettes, granulés, extrudés, agglomérés, et autres, de section circulaire, ovale, trilobée ou multilobée, pleine ou creuse). Les catalyseurs de type billes, pastilles et autres, présentent l'avantage de pouvoir être séparés, ultérieurement, du milieu de réaction, très rapidement par simple décantation. Les catalyseurs de type pulvérulent nécessitent généralement, pour leur séparation, une étape de filtration.

Bien entendu, tous les catalyseurs précités sont choisis avec une surface spécifique convenant pour l'application considérée. Dans la pratique, on peut mettre en oeuvre un carbure de tungstène dont la surface spécifique mesurée selon la méthode BET (Brunauer, Emmett et Teller) peut varier d' un dixième à plusieurs centaines, voire quelques milliers de mètres carrés par gramme et en général de 1 à 500 $m^2/g$.

A cet effet, on pourra utiliser soit des carbures de tungstène disponibles dans le commerce, soit des carbures de tungstène que l'on aura synthétisés selon tout procédé connu en soi. A titre d'exemple, des carbures de tungstène à hautes surfaces spécifiques peuvent être fabriqués selon le procédé décrit dans la demande de brevet PCT/FR 90/00204.

On préfère les carbures de tungstène dont le rapport tungstène/ carbone est aux alentours de 1 et désignés par WC.

Avantageusement, le procédé s'applique aux dérivés nitrés répondant à la formule (I) :

$$(Z)q\ (Y)p\ (X)n - Ar - (NO_m)x$$

dans laquelle

- Ar représente un reste aromatique mono ou polycyclique, soit homo soit hétérocyclique, éventuellement substitué par un groupe alcoyle contenant de préférence 1 à 4 atomes de carbone, aralcoyle, alcényle, ou un groupe fonctionne tel que hydroxyle, trifluorométhyle, nitrile, acide, ester, cétone, acide a insaturé, éther, hétérocycle ;
- X, Y et Z représentent un halogène, avantageusement choisi parmi le fluor, le chlore et le brome ;
- x = 1, 2 voire 3 ;
- n, p et q représentent un nombre entier compris entre 0 et 5 ;
  la somme n+p+q pouvant être égale ou supérieure à 0 ;
  m est choisi entre 1 et 2, le dit composé ayant au plus 50 atomes de carbone.

De préférence Ar représente un radical aromatique monocyclique et, X et Y représentent le chlore et/ou le fluor et la somme n+p est égale ou supérieure à 1 et inférieure ou égale à 3 et q est égal à zéro.

Lorsqu'il est utilisé pour une amidation au moins partielle de l'aniline, en cours de formation, le procédé vise, outre ceux spécifiés ci-dessus dans la formule I, ceux dont la formule suit et où Ar est : $(Ed)_r$-Ar' :

$$(Ed)_r\text{-}Ar' - (NO_m)x \qquad \text{formule (II)}$$

où Ed représente un ou plusieurs groupes, semblables ou différents, choisi(s) parmi les alcoyles contenant de préférence 1 à 10 atomes de carbone, aralcoyle, alcényle, ou un groupe fonctionnel tel que hydroxyle, trifluorométhyle, nitrile, acide, ester, cétone, acide $\alpha$ insaturé, éther, hétérocycle ;

où r est un entier compris entre 0 et 3, avantageusement choisi parmi 0, 1, ou 2

où m est un entier compris entre 1 et 2 (c'est-à-dire 1 ou 2)

Ar et x ont les valeurs indiquées ci-dessus.

Ainsi Ar' est un reste aromatique mono- ou polycyclique, soit homo- soit hétérocyclique,

Tant pour Ar que pour Ar' le nombre de cycles est de préférence au plus égal à 5 de préférence à 3

La réaction est particulièrement intéressante pour les molécules où les Ed sont des alcoxyles, des acyloxyles, et/ou des hydroxyles

En fait les différents Ed ne modifient pas significativement la faisabilité de la réaction mais ces groupes sont intéressants car le carbures de tungstène permet une sélectivité insoupçonnée de l'hydrogénation du groupe nitro vis à vis des groupes Ed. Il faut souligner aussi que lorsque les groupes sont électrodonneurs, la réactivité de la fonction aniline est exacerbée ; ce qui dans la plupart des cas conduit à de nombreux sous produits notamment des lourds. Une remarque similaire peut être faite pour les polynitrés sur un même noyau qui conduisent à des polyanilines réputées extrêmement réactives.

Le nombre de carbone de chacun des groupes Ed est en général au plus égal à 10 de préférence à 6.

Enfin on ne s'écartera pas de l'invention en hydrogénant des dérivés portant plus de trois fonctions nitrées à condition que l'on ait au plus trois fonctions nitrées par noyau.

Quoique la palette des dérivés traitables de cette manière soit extrêmement vaste, il est avantageux que ces composés soient liquides dans les conditions opératoires ou bien soient solubles dans le milieu réactionnel.

Ainsi il est plus aisé de manipuler des composé dont le nombre total de carbone soit au plus égal à 50 de préférence à 30.

Ce procédé (et donc ce réactif) convient bien pour la synthèse des anilines et surtout polyanilines qui sont les matières premières des mono- di- et polyisocyanates tel que par exemple le toluène diamine 2,4 origine de la synthèse du toluène diisocyanate (TDI), puis de nombreux polyuréthannes.

Lorsque l'on désire faire la réaction d'amidation, on utilise comme solvant, un milieu contenant l'acide dont on désire faire l'amide. Il peut contenir outre cet acide de l'eau et un solvant inerte de préférence polaire.

Parmi les acides, on peut citer les acides sulfuriques, sulfoniques, phosphoriques et surtout carboxyliques. Il est préférable que le point d'ébullition de ces acides soit supérieur à 150° environ, si cela n'est pas le cas, il faudra accepter de travailler sous pression partielle, dudit acide supérieur à une atmosphère ($10^5$ Pascal). La réaction est bien adaptée aux acides, de préférence monoacides, carboxyliques, dont le nombre de carbones est compris entre 1 et 30, de préférence entre 2 et 20. Cette réaction est particulièrement intéressante pour les acides de bas poids moléculaire notamment l'acide acétique. Cette réaction marche particulièrement bien lorsque les positions para- ou quasi-para sont occupées par un substituant notamment donneur. Ce substituant peut en particulier, être une fonction phénol ou une fonction dérivé, ester, éther.

A titre indicatif, on peut en principe mettre en oeuvre la réaction d'amidation à une température comprise entre 0 et 300°C (1 chiffre significatif).

En général, cette réaction d'amidation a lieu à haute température, c'est-à-dire à une température significativement supérieure à 100°C ; la température à partir de laquelle la réaction d'amidation commence, dépend des substrats, des acides et de la concentration en acide. Elle est facilement déterminable par l'homme de l'art, au moyen d'essais de routine, dès lors qu'il connaît l'existence de cette amidation dans des conditions non-concomitantes de l'hydrogénation.

Cette amidation est en général quasi complète pour des températures supérieures ou égales à 150°C.

Aussi choisit on de préférence une température de réaction comprise entre 150°C et 250°C.

Cette réaction est à la fois très intéressante, au plan économique, car elle permet d'utiliser des réactifs peu chers comme les acides au lieu des divers anhydrides (y compris les anhydrides mixtes obtenu par élimination d'eau entre les acides halohydriques et les acides oxygénés, anhydrides qui sont en fait des chlorures d'acide) et très surprenantes au plan scientifique. En effet, lors des hydrogénations conduites auparavant, on était contraint de travailler à des températures relativement basses, en général au plus égales à 100°C pour éviter la formation de nombreux sous-produits difficiles à séparer ultérieurement d'avec le produit désiré.

Il est possible d'utiliser en lieu et place de l'acide libre, des réactifs (anhydride symétrique ou mixte, ester), qui dans les conditions opératoires libèrent l'acide dont on désire faire l'anilide.

De manière totalement surprenante, la présence de carbure de tungstène, d'une part prévient la formation de ces sous-produits, et d'autre part semble faciliter la réaction d'amidation.

Bien entendu, pour obtenir un rendement satisfaisant en anilide, il est souhaitable d'utiliser de l'acide en excès stoechiométrique (il n'y a pas de limite supérieure, sinon économique, mais on peut mentionner que, dans le cas d'un procédé discontinu un excès en début de réaction de 0,1 à 4 fois, de préférence de 0,5 à 3 fois la quantité stoechiométrique donne de bon résultat ; dans le cas d'un procédé continu on préfère des excès plus élevé que la plus basse des valeurs ci-dessus)

La quantité d'eau peut varier entre 0, de préférence 10, à 50 % en volume de l'acide utilisé.

Un autre but de la présente invention, est de fournir un nouveau réactif d'hydrogénation, à base de carbure de tungstène, dont les caractéristiques cinétiques soient suffisamment améliorées par rapport au carbure de tungstène.

Ce but est atteint, par un réactif caractérisé par le fait qu'il comporte les espèces suivantes :

- le matériau selon l'invention ;
- une phase liquide, telle que décrite ci-dessus
- de l'hydrogène à une pression au moins égale à environ 2 atmosphères avantageusement à environ 5 atmosphères, de préférence à environ 10 atmosphères.

Les exemples non limitatifs suivants illustrent l'invention.

Dans les exemples suivants, le carbure de tungstène présente une surface spécifique d'environ 1 $m^2$/g.

En ce qui concerne la pression partielle d'hydrogène, il convient de rappeler que l'on a pratiqué, selon les techniques usuelles en la matière, c'est-à-dire que le réacteur dans lequel la réaction est réalisée est relié à une bouteille d'hydrogène, au moyen d'un dispositif anti-retour régulant la pression dans le réacteur à la valeur affichée. La pression partielle de l'hydrogène, à la température réactionnelle, est donc la pression affichée diminuée de la pression autogène du milieu réactionnel.

Il convient de rappeler, que la présente invention n'est pas limitée à l'emploi d'hydrogène pur ,il peut être utilisé sous la forme d'un mélange de gaz dès lors que les gaz avec lesquels il est mélangé, sont sensiblement inertes dans les conditions réactionelles

Sa surprenante faible sensibilité aux poisons sulfurés, prédispose le réactif selon l'invention, à l'utilisation de gaz issus de la gazéification des dérivés carbonés minéraux comme les charbons.citons en particulier les gaz à l'eau. En ce qui concerne les rendement ,on peut rappeler les abréviations suivantes.

$$\text{TT (taux de transformation)} = \frac{\text{Q.S.I. - Q.S.R.}}{\text{Q.S.I.}}$$

$$\text{RT (rendement sur produit transformé )} = \frac{\text{Q.D.D.F.}}{\text{Q.S.I. - Q.S.R.}}$$

$$\text{RR (rendement sur produit introduit)} = \frac{\text{Q.D.D.F.}}{\text{Q.S.I.}}$$

Q.S.I.= Quantité de Substrat Introduit (exprimée en mole)
Q.S.R.=-Quantité de Substrat Récupéré en fin de réaction (exprimée en mole)
Q.D.D.F. = Quantité de Dérivé Désiré Formé (exprimée en mole)

Exemple N°1 Hydrogénation du nitrobenzène

Dans une ampoule en verre de 35 ml, on introduit 0,5 g de nitrobenzène, on ajoute 15 ml de EtOH (alcool éthylique) et 0,47 g de carbure de tungstène. On introduit l'ampoule dans un autoclave de 125 ml en inox. On purge avec 2x10 bar d'azote puis 2x20 bar d'hydrogène. On met sous pression d'hydrogène 20 bar et on chauffe à 100°C en agitant. On maintient dans ces conditions pendant 4 heures. On refroidit l'autoclave dans un bain d'eau. On soutire la phase organique.

Par analyse en chromatographie en phase gazeuse (CPG), on obtient un TT de 100 % et une sélectivité de 95 %.

Le système catalytique est recyclable sans perte d'activité.

Exemple N°2 Hydrogénation du nitrobenzène avec transposition de Bamberger

Dans une ampoule en verre de 35 ml, on introduit 1,0 g de nitrobenzène et 8 ml d'acide sulfurique à 40 %. On ajoute 0,97 g de carbure de tungstène. On introduit l'ampoule en verre dans un autoclave de 125 ml. On purge avec 2x 5 bar d'azote puis 3 x 5 bar d'hydrogène.

On met sous 5 bar d'hydrogène. La pression est maintenue constante pendant toute la durée de la réaction. On chauffe à 115°C en agitant. Après 5h45 de réaction, l'autoclave est refroidit par un bain d'eau. Par analyse du CPG milieu réactionnel après traitement, on obtient un TT de 99,8 % et 58% de paminophénol.

Le système catalytique est recyclable sans perte d'activité.

Exemple N°3 Hydrogénation du dichloro-3,4-nitrobenzène

Dans un autoclave de 125 ml, on charge 10 g de dichloro-3,4 nitrobenzène, on ajoute 5,1 g de carbure de tungstène et 40 ml de méthanol.

On purge avec 2x 5 bar d'azote et 2 x 20 bar d'hydrogène. On met sous 20 bar d'hydrogène, on agite et on chauffe

à 110°C. La pression est maintenue constante.

Après 4 heures de réaction, on purge l'autoclave avec 2 x 10 bar d'azote lorsque celui-ci est froid. On filtre le milieu réactionnel.

Par analyse CPG (Chromatographie en Phase Gazeuse), on détermine le TT qui est égal à 100. Sa sélectivité en dichloro-3,4 aniline est supérieure à 99 %. Par dosage polarographique on détecte la présence de chlorure qui correspond à un TT molaire au plus égal à 0,05 %.

Exemple N°4 Hydrogénation du p nitrophénol

Dans une ampoule, en verre de 35 ml, on introduit 0,55 g de p nitrophénol, 15 ml de méthanol et 0,46 g de carbure de tungstène.

On introduit l'ampoule en verre dans l'autoclave de 125 ml en inox. On purge avec 2 x 10 bar d'azote puis 2 x 20 bar d'hydrogène. On met sous pression d'hydrogène 20 bar et on chauffe à 100°C en agitant. On maintient dans ces conditions pendant 4 heures.

On refroidit dans un bain d'eau. On soutire la phase organique, que l'on dose par CPG.

On obtient TT 59 % et une sélectivité de 99 %.

Le système catalytique est recyclable sans perte d'activité.

Exemple N°5 Rôle du solvant dans l'hydrogénation du p nitrophénol

Dans un autoclave de 30 ml, on charge 0,55g de p nitrophénol et 0,39g WC. On ajoute 10 ml de solvant eau-acide acétique en proportions variables.

On purge à l'azote 2x5 bar puis à l'hydrogène 2x20 bar. On met sous 20 bar d'hydrogène et on chauffe à une température de 100°C, en agitant et en maintenant la pression à 20 bar. Après 4 heures de réaction, on analyse les essais par CPG.

| SOLVANTS | | RENDEMENT EN AMINOPHENOL PAR RAPPORT AU PARA-NITRO PHENOL | |
|---|---|---|---|
| ACOH ml | $H_2O$ ml | TT | RR |
| 10 | 0 | 8 | 6 % |
| 9 | 1 | 39 | 39 % |
| 8 | 2 | 69 | 69 % |
| 6 | 4 | 87 | 85 % |
| 5 | 5 | 98 | 98 % |
| 2 | 8 | 99 | 98 % |

Exemple N°6 Rôle du solvant dans l'hydrogénation du p nitrophénol dans le cas du catalyseur a base de palladium

par comparaison en utilisant un catalyseur a base de palladium : 9mg 3% Pd/C.

| SOLVANTS | | RENDEMENT EN AMINOPHENOL PAR RAPPORT AU PARA-NITRO PHENOL | |
|---|---|---|---|
| ACOH ml | $H_2O$ ml | TT | RR |
| | | | |
| 10 | 0 | 100% | 54% |

Exemple N°7 Réaction en phase vapeur

Dans un réacteur en verre de 20 mm, on introduit 5 ml de quartz 1 ml de WC et 5 ml de quartz. Le réacteur est chauffé par un four électrique à 450°C pendant 1 heure, en balayant le lit catalytique par un courant d'hydrogène à $2lh^{-1}$. La température est ensuite ramenée à 240° C et toujours avec un courant de $2lh^{-1}$, on introduit à l'aide d'un pousse seringue le nitrobenzène à un débit de 0,5 $mlh^{-1}$.

Après 3 heures de réaction, l'analyse CPG donne le taux de conversion TT et le rendement réel RR.

TT = 48 %
RR = 39 %

Exemple N°8 Préparation d'APAP par coréaction hydrogénation acylation dans l'acide acétique catalysée par le Pd/C

Dans une ampoule de verre de 35 ml, on introduit 7,5g de p nitrophénol, 15ml d'un mélange acétique-eau de rapport 80%/20% respectivement. On introduit 15 mg de Pd/C à 3%. On introduit l'ampoule de verre dans un autoclave de 125 ml. On ferme l'autoclave. On purge avec 2x10 bar d'azote puis avec 2x10 bar d'hydrogène.

On place ensuite celui-ci, sous 20 bar de pression, et on chauffe à 150°C sous agitation. Après la fin de l'absorption d'hydrogène, on refroidit à température ambiante. On analyse le milieu réactionnel par HPLC. La conversion est de 17 % et le catalyseur est empoisonné.

Exemple N°9 Préparation d'APAP par coréaction hydrogénation, acylation dans l'acide acétique catalysée par le carbure de tungstène.

Dans une ampoule de verre de 35 ml, on introduit 7,5g de p nitrophénol, 15ml d'un mélange $CH_3COOH/H_2O$ à une teneur de 80% d'acide acétique et 20% d'eau. On introduit 3g de carbure de tungstène. L'ampoule de verre ouverte, est introduite dans un autoclave de 125 ml. On ferme celui-ci, on purge avec 2x10 bar d'azote puis avec 2x10 bar d'hydrogène. On place ensuite l'autoclave sous 20 bar d'hydrogène et on chauffe à 150°C sous agitation. La pression de l'autoclave est maintenue à 20 bar pendant toute la durée de réaction.

Après la fin d'absorption d'hydrogène, on refroidit à température ambiante. La conversion est totale et on dose par HPLC 99,5% d'APAP. Le carbure de tungstène est recyclé sous perte d'activité.

Exemple N°10 Réduction du chloro-5 fluoro-2 nitrobenzène

Dans une ampoule de verre de 35 ml, on introduit 10g de chloro-5 fluoro-2 nitrobenzène et 10 ml de mélange eau-méthanol dans le rapport 2/8. On introduit ensuite 2,5g de carbure de tungstène. On charge l'ampoule de verre ouverte dans un autoclave de 125 ml. On purge celui-ci avec 2x10 bar d'azote puis avec 2x10 bar d'hydrogène. On place ensuite le réacteur sous 20 bar d'hydrogène, on agite et on chauffe à 120°C. On maintient pendant toute la durée de la réaction, la pression de 20 bar dans l'autoclave. Après 4 heures de réaction, la consommation d'hydrogène cesse.

On maintient encore, pendant 1 heure, dans ces conditions. L'analyse CPG nous montre que la conversion est totale et que le rendement en chloro-5 fluoro-2 aniline est supérieur à 99,8%. L'hydrodéshalogénation est inférieure à 0,2% mesuré par ionométrie.

Exemple N°11 Réduction du dichloro-2,3 nitrobenzène

Dans une ampoule de verre de 35 ml, on introduit 10g de dichloro-2,3 nitrobenzène et 10 ml d'un mélange eau méthanol de rapport 2/8. On ajoute 2,5g de carbure de tungstène. On introduit l'ampoule de verre dans un autoclave de 125 ml. On ferme celui-ci et on purge par 2x10 bar d'azote puis par 2x10 bar d'hydrogène. On place ensuite l'autoclave sous 20 bar d'hydrogène et on chauffe en agitant à 120°C. On maintient la pression de 20 bar sur l'autoclave.

Après 3 heures de réaction, la consommation d'hydrogène cesse. On maintient encore 1 heure dans ces conditions de température et de pression. Par dosage CPG, on montre que la conversion est totale et le rendement en 2,3-dichloroaniline est supérieur à 99,5 %. L'hydrodéchloration est inférieure à 0,2%. Le catalyseur est recyclable sans perte d'activité.

Exemple N°12 Hydrogénation acylation dans l'acide acétique du chloro 3 fluoro 4 nitrobenzène.

Dans une ampoule en verre de 35 ml. On introduit 10g de chloro 3 fluoro 4 nitrobenzène et 10ml. D'un mélange acide acétique / eau 90/10. On ajoute 2,5 de carbure de tungstène. L'ampoule de verre est introduite dans un autoclave de 125ml.

On forme celui-ci ou purge avec 2 x 10 bar de $N_2$ et ensuite par 2x10 bar d'hydrogène. On place ensuite l'autoclave sous 20 bar d'hydrogène et on chauffe à 150°C en agitant.

La pression d'hydrogène est maintenue à 20 bar pendant toute la durée de réaction.

Après la fin d'absorption d'hydrogène, on refroidit.

La conversion est totale et le rendement en 3 chloro 4 fluoro acétanilide est de 97 %.

Exemple N°13 : Hydrogénation acylation dans l'acide acétique du dichloro 3, 4 nitrobenzène.

Dans une ampoule en verre de 35 ml., on introduit 10 g de dichloro 3,4 nitrobenzène et 10 ml. D'un mélange acide acétique/eau 90/10. On ajoute 2,5 g de carbure de tungstène.

L'ampoule de verre est introduite dans un autoclave de 125 ml.. On ferme celui-ci, on purge avec 2x10 bar d'azote puis 2x10 bar d'hydrogène.

On place ensuite l'autoclave sous 20 bar d'hydrogène et on chauffe à 150°C en agitant.La pression de l'autoclave est maintenue à 20 bar pendant toute la durée de réaction.

Après la fin d'absorption de l'hydrogène, on refroidit. La conversion est totale et le rendement en N acetyl-dichloro 3,4 aniline est de 98 %.

Exemple N°14 : Hydrogénation du dinitro 2, 4 toluène.

Dans un réacteur SOTELEM de 750 ml., on introduit 200 ml. d'eau et 2,5 g de carbure de tungstène.

L'ampoule de verre est introduite dans un autoclave de 125 ml.. On ferme celui-ci, on le purge avec 2x10 bar d'azote puis 2x10 bar d'hydrogène.

On place ensuite l'autoclave sous 20 bar d'hydrogène et on chauffe à 185°C en agitant. On injecte alors en 70mn une solution composée de 26 g de dinitro 2,4 toluène et 100 ml de diglyme.

La pression totale de l'autoclave est maintenue à 90 bar pendant toute la durée de réaction.

La consommation d'hydrogène mesurée par une différence de pression dans une réserve de volume connu. Dès la fin de l'injection la consomation d'hydrogène cesse.

Un dosage en CPG (chromatographie en phase gazeuse) donne :

TT = 100 %
RR = 99 % en Toluène diamine 2,4

**Revendications**

1. Procédé pour l'hydrogénation de dérivé, nitré ou nitroso en groupe aminé où ledit dérivé est hydrogéné en présence de carbure de tungstène, caractérisé par le fait que l'hydrogénation se fait en phase liquide, que la pression partielle d'hydrogène est au moins égale à cinq atmosphères ($5.10^5$ Pascals) et au plus égale à deux cents atmosphères ($2.10^7$ Pascals) et que ledit dérivé, nitré ou nitroso est aromatique et par le fait que ledit dérivé azoté répond à la formule (I) :

$$(Z)q\ (Y)p\ (X)n - Ar - (NO_m)x$$

dans laquelle :

- Ar représente un reste aromatique, éventuellement substitué par un groupe alcoyle contenant de préférence 1 à 4 atomes de carbone, aralcoyle, alcényle, ou un groupe fonctionnel tel que hydroxyle, trifluorométhyle, nitrile, acide, ester, cétone, acide $\alpha$ insaturé, éther, hétérocycle ;
- X, Y et Z représentent un halogène, avantageusement choisi parmi le fluor, le chlore et le brome ;
- x = 1, 2 voire 3 ;
- n, p et q représentent un nombre entier compris entre 0 et 5 ;
  la somme n+p+q pouvant être égale ou supérieure à 0 et m étant choisi entre 1 et 2, le dit composé ayant au plus 50 atomes de carbone.

2. Procédé selon la revendication 1, caractérisé par le fait que le dit carbure de tungstène est activé en le soumettant à une température au moins égale a 50°C, avantageusement à 100°C et à une pression d'hydrogène au moins égale à 2 atmosphères avantageusement à 5 atmosphères.

3. Procédé selon la revendication 1, caractérisé par le fait que le dit carbure de tungstène est activé en le soumettant

à l'une des conditions suivantes :

- températures supérieures ou égales à 150°C pression partielle d'hydrogène au moins égale à 2 atmosphères.
- à des pressions supérieures à 10 de préférence à 20 atmosphères
- à une température au moins égale à 100°C et à une pression supérieure à 10 atmosphères.

**4.** Procédé selon la revendication 3, caractérisé par le fait que le dit carbure de tungstène est activé en présence d'un milieu liquide acide.

**5.** Procédé selon la revendication 4, caractérisé par le fait que Ar représente un radical aromatique monocyclique et X et Y représentent le chlore et/ou le fluor et la somme n+p est égale ou supérieure à 1 et inférieure ou égale à 3.

**6.** Procédé selon l'une des revendications 4 et 5, caractérisé par le fait que q est égal à zéro.

**7.** Procédé selon l'une des revendications 1 à 6, caractérisé par le fait que la quantité de catalyseur à utiliser varie entre 0,01 % et 50 % en poids par rapport à la quantité de substrat engagée.

**8.** Procédé selon l'une des revendications 1 à 7, caractérisé par le fait que ladite réaction est menée en présence d'un acide fort dans les conditions d'obtention de la transposition de Bamberger.

**9.** Procédé selon la revendication 8, caractérisé par le fait que ledit acide fort est choisi parmi les acides phosphoriques, l'acide sulfurique, les acides halohydriques et leurs mélanges.

**10.** Procédé selon les revendications 8 et 9, caractérisé par le fait que ledit acide fort est choisi parmi l'acide chlorhydrique ou l'acide bromhydrique.

**11.** Procédé selon les revendications 1 à 10, caractérisé par le fait que la réaction est réalisée en l'absence de solvant ou dans un solvant choisi parmi les alcools, les dérivés aromatiques et leurs mélanges, avantageusement le solvant est choisi parmi les solvants protiques et de préférence parmi l'eau et les alcools, avantageusement le méthanol, et les mélanges en contenant.

**12.** Procédé selon l'une des revendications 1 à 11, caractérisé par le fait que la réaction est menée a une température comprise entre l'ambiante et la température d'ébullition du mélange réactionnel dans les conditions opératoires, avantageusement entre 20°C et 200°C, et de préférence entre 50 et 150°C.

**13.** Procédé selon l'une des revendications 1 à 12, caractérisé par le fait que la réaction est conduite sous une pression partielle en hydrogène comprise entre 5 à 100 bar et de préférence de 5 à 20 bar.

**14.** Procédé selon l'une des revendications 4 à 13, caractérisé par le fait que la réaction est conduite en présence d'un acide susceptible de former des anilides et à une température supérieure à 100°C, avantageusement lorsque l'acide est un acide carboxylique, à une température au moins égale à environ 150°C.

**15.** Procédé selon l'une des revendications 1 à 14, caractérisé par le fait que Ar est $(Ed)_r$-Ar' où Ed représente un ou plusieurs groupes, semblables ou différents, choisi(s) parmi les alcoyles contenant de préférence 1 à 10 atomes de carbone, aralcoyle, alcényle, ou un groupe fonctionnel tel que hydroxyle, trifluorométhyle, nitrile, acide, ester, cétone, acide $\alpha$ insaturé, éther, hétérocycle et où r est un entier compris entre 0 et 3, avantageusement choisi parmi 0, 1, ou 2.

**16.** Procédé selon la revendication 15, caractérisé par le fait que ledit dérivé azoté répond à la formule (II) :

$$(Ed)_r\text{-}Ar' - (NO_m)x \qquad \text{formule (II)}$$

où

Ed représente un ou plusieurs groupes, semblables ou différents, choisi(s) parmi les alcoyles contenant de préférence 1 à 10 atomes de carbone, aralcoyle, alcényle, ou un groupe fonctionnel tel que hydroxyle, trifluorométhyle, nitrile, acide, ester, cétona, acide $\alpha$ insaturé, éther, hétérocycle ;
où

r est un entier compris entre 0 et 3, avantageusement choisi parmi 0, 1, ou 2 ;
où
m est un entier compris entre 1 et 2 ; où

- Ar' représente un reste aromatique, éventuellement substitué par un groupe alcoyle contenant de préférence 1 à 4 atomes de carbone, aralcoyle, alcényle, ou un groupe fonctionnel tel que hydroxyle, trifluorométhyle, nitrile, acide, ester, cétone, acide a insaturé, éther, hétérocycle ;
  où
- x = 1, 2 voire 3 ;

**17.** Procédé selon l'une des revendications 4 à 16, caractérisé par le fait que le nombre de cycles de Ar est au plus égal à 5 de préférence à 3.

**18.** Procédé selon l'une des revendications 16 et 17, caractérisé par le fait que le nombre de cycles de Ar' est au plus égal à 5 de préférence à 3.

**19.** Procédé selon l'une des revendications 16 à 18, caractérisé par le fait que les Ed sont des alcoxyles, des acyloxyles, et/ou des hydroxyles.

**20.** Procédé selon l'une des revendications 1 à 19, caractérisé par le fait que ledit dérivé azoté orésente au plus 50, de préférence au plus 30 atomes de carbone.

**21.** Réactif d'hydrogénation, à base de carbure de tungstène, caractérisé par le fait qu'il comporte les espèces suivantes :

- un matériau susceptible d'être obtenu en soumettant du carbure de tungstène à un traitement d'hydrogénation avantageusement en phase liquide, à une température au moins égale à 50°C avantageusement à 100°C à une pression au moins égale à 5 atmosphères, de préférence à 10 atmosphères.;

- une phase liquide ;

- de l'hydrogène à une pression au moins égale à 2 atmosphères avantageusement à 5 atmosphères, de préférence à 10 atmosphères et au plus égale à à deux cents atmosphères ($2.10^7$ Pascals).

**22.** Réactif selon la revendication 21, caractérisé par le fait que ladite phase liquide comporte un solvant choisi parmi les alcools, les dérivés aromatiques et leurs mélanges, avantageusement le solvant est choisi parmi les solvants protiques et de préférence parmi l'eau et les alcools, avantageusement le méthanol, et les mélanges en contenant.

**23.** Réactif selon les revendications 21 et 22, caractérisé par le fait que ladite phase liquide présente une teneur en acide au moins égale à 0,1 N, avantageusement à 1 N.

**24.** Réactif selon les revendications 21 à 23, caractérisé par le fait que ladite phase liquide comporte un acide fort choisi parmi les acides sulfuriques, sulfoniques, phosphoriques, carboxyliques, halohydriques et leurs mélanges.

**25.** Réactif selon les revendications 21 à 24, caractérisé par le fait que ladite phase liquide comporte un acide fort utilisable pour la transposition de Bamberger.

**26.** Réactif selon la revendication 25, caractérisé par le fait que ladite phase liquide comporte un acide fort choisi parmi les acides phosphoriques, l'acide sulfurique, les acides halohydriques et leurs mélanges.

**27.** Réactif selon les revendications 25 à 26, caractérisé par le fait que ladite phase liquide comporte un acide fort choisi parmi l'acide chlorhydrique ou l'acide bromhydrique.

**28.** Réactif selon les revendications 21 à 27, caractérisé par le fait que ladite phase liquide comporte un acide carboxylique.

**29.** Réactif selon la revendication 28, caractérisé par le fait que ledit acide carboxylique est un monoacide de 1 a 20 atomes de carbone.

**30.** Catalyseur caractérisé par le fait qu'il est constitué au moins en partie d'un matériau susceptible d'être obtenu en soumettant du carbure de tungstène à un traitement d'hydrogénation avantageusement en phase liquide, à une température au moins égale à 50°C avantageusement à 100°C à une pression au moins égale à 2 atmosphères avantageusement à 5 atmosphères, de préférence à 10 atmosphères.

**31.** Catalyseur selon la revendication 30 caractérisé par le fait que la surface spécifique dudit matériau est comprise entre un 1/10° et 1000 $m^2/g$, plus généralement de 1 à 500 $m^2/g$.

**Claims**

**1.** Process for the hydrogenation of a nitro or nitroso derivative to an amino group, where the said derivative is hydrogenated in the presence of tungsten carbide, characterized in that the hydrogenation is carried out in the liquid phase, that the hydrogen partial pressure is at least equal to five atmospheres ($5 \times 10^5$ pascals) and at most equal to two hundred atmospheres ($2 \times 10^7$ pascals) and that the said nitro or nitroso derivative is aromatic and in that the said nitrogenous derivative corresponds to the formula (I):

$$(Z)_q(Y)_p(X)_n\text{-Ar-}(NO_m)_x$$

in which:

- Ar represents an aromatic residue, optionally substituted by an alkyl, preferably containing 1 to 4 carbon atoms, aralkyl or alkenyl group or a functional group, such as hydroxyl, trifluoromethyl, nitrile, acid, ester, ketone, $\alpha$-unsaturated acid, ether or heterocycle;
- X, Y and Z represent a halogen, advantageously chosen from fluorine, chlorine and bromine;
- x = 1, 2 or 3;
- n, p and q represent an integer of between 0 and 5;
  it being possible for the sum n+p+q to be equal to or greater than 0 and m being chosen between 1 and 2, the said compound having at most 50 carbon atoms.

**2.** Process according to claim 1, characterized in that the said tungsten carbide is activated by subjecting it to a temperature at least equal to 50°C, advantageously to 100°C, and to a hydrogen pressure at least equal to 2 atmospheres, advantageously to 5 atmospheres.

**3.** Process according to claim 1, characterized in that the said tungsten carbide is activated by subjecting it to one of the following conditions:

- to temperatures greater than or equal to 150°C, hydrogen partial pressure at least equal to 2 atmospheres,
- to pressures greater than 10, preferably than 20 atmospheres,
- to a temperature at least equal to 100°C and to a pressure greater than 10 atmospheres.

**4.** Process according to claim 3, characterized in that the said tungsten carbide is activated in the presence of an acidic liquid medium.

**5.** Process according to claim 4, characterized in that Ar represents a monocyclic aromatic radical and X and Y represent chlorine and/or fluorine and the sum n+p is equal to or greater than 1 and less than or equal to 3.

**6.** Process according to either of claims 4 and 5, characterized in that q is equal to zero.

**7.** Process according to one of claims 1 to 6, characterized in that the amount of catalyst to be used varies between 0.01% and 50% by weight with respect to the amount of substrate involved.

**8.** Process according to one of claims 1 to 7, characterized in that the said reaction is carried out in the presence of a strong acid under conditions for obtaining the Bamberger rearrangement.

**9.** Process according to claim 8, characterized in that the said strong acid is chosen from phosphoric acids, sulphuric acid, hydrohalic acids and their mixtures.

**10.** Process according to claims 8 and 9, characterized in that the said strong acid is chosen from hydrochloric acid or

hydrobromic acid.

11. Process according to claims 1 to 10, characterized in that the reaction is carried out in the absence of solvent or in a solvent chosen from alcohols, aromatic derivatives and their mixtures; the solvent is advantageously chosen from protic solvents and preferably from water and alcohols, advantageously methanol, and the mixtures containing them.

12. Process according to one of claims 1 to 11, characterized in that the reaction is carried out at a temperature between ambient temperature and the boiling temperature of the reaction mixture under the operating conditions, advantageously between 20°C and 200°C, preferably between 50 and 150°C.

13. Process according to one of claims 1 to 12, characterized in that the reaction is carried out under a hydrogen partial pressure of between 5 and 100 bar and preferably from 5 to 20 bar.

14. Process according to one of claims 4 to 13, characterized in that the reaction is carried out in the presence of an acid capable of forming anilides and at a temperature greater than 100°C, advantageously when the acid is a carboxylic acid, at a temperature at least equal to approximately 150°C.

15. Process according to one of claims 1 to 14, characterized in that Ar is $(Ed)_r$-Ar', where Ed represents one or more groups, which are alike or different, chosen from alkyls, preferably containing 1 to 10 carbon atoms, aralkyl, alkenyl or a functional group, such as hydroxyl, trifluoromethyl, nitrile, acid, ester, ketone, α-unsaturated acid, ether or heterocycle and where r is an integer of between 0 and 3, advantageously chosen from 0, 1 or 2.

16. Process according to claim 15, characterized in that the said nitrogenous derivative corresponds to the formula (II):

$$(Ed)_r\text{-}Ar'\text{-}(NO_m)_x \qquad \text{formula (II)}$$

where

Ed represents one or more groups, which are alike or different, chosen from alkyls, preferably containing 1 to 10 carbon atoms, aralkyl, alkenyl or a functional group, such as hydroxyl, trifluoromethyl, nitrile, acid, ester, ketone, α-unsaturated acid, ether or heterocycle;
where
r is an integer of between 0 and 3, advantageously chosen from 0, 1 or 2;
where
m is an integer of between 1 and 2;
where
Ar' represents an aromatic residue, optionally substituted by an alkyl, preferably containing 1 to 4 carbon atoms, aralkyl or alkenyl group or a functional group, such as hydroxyl, trifluoromethyl, nitrile, acid, ester, ketone, α-unsaturated acid, ether or heterocycle; where
x = 1, 2 or 3.

17. Process according to one of claims 4 to 16, characterized in that the number of Ar rings is at most equal to 5, preferably to 3.

18. Process according to either of claims 16 and 17, characterized in that the number of Ar' rings is at most equal to 5, preferably to 3.

19. Process according to one of claims 16 to 18, characterized in that the Ed groups are alkoxys, acyloxys and/or hydroxyls.

20. Process according to one of claims 1 to 19, characterized in that the said nitrogenous derivative exhibits at most 50, preferably at most 30, carbon atoms.

21. Hydrogenation reagent based on tungsten carbide, characterized in that it contains the following species:

- a material capable of being obtained by subjecting tungsten carbide to a hydrogenation treatment advantageously in the liquid phase, at a temperature at least equal to 50°C, advantageously to 100°C, at a pressure at

least equal to 5 atmospheres, preferably to 10 atmospheres;

- a liquid phase;
- hydrogen at a pressure at least equal to 2 atmospheres, advantageously to 5 atmospheres, preferably to 10 atmospheres, and at most equal to two hundred atmospheres (2 x $10^7$ pascals).

**22.** Reagent according to claim 21, characterized in that the said liquid phase contains a solvent chosen from alcohols, aromatic derivatives and their mixtures; the solvent is advantageously chosen from protic solvents and preferably from water and alcohols, advantageously methanol, and the mixtures containing them.

**23.** Reagent according to claims 21 and 22, characterized in that the said liquid phase exhibits an acid content at least equal to 0.1N, advantageously to 1N.

**24.** Reagent according to claims 21 to 23, characterized in that the said liquid phase contains a strong acid chosen from sulphuric, sulphonic, phosphoric, carboxylic and hydrohalic acids and their mixtures.

**25.** Reagent according to claims 21 to 24, characterized in that the said liquid phase contains a strong acid which can be used for the Bamberger rearrangement.

**26.** Reagent according to claim 25, characterized in that the said liquid phase contains a strong acid chosen from phosphoric acids, sulphuric acid and hydrohalic acids and their mixtures.

**27.** Reagent according to claims 25 and 26, characterized in that the said liquid phase contains a strong acid chosen from hydrochloric acid or hydrobromic acid.

**28.** Reagent according to claims 21 to 27, characterized in that the said liquid phase contains a carboxylic acid.

**29.** Reagent according to claim 28, characterized in that the said carboxylic acid is a monoacid containing 1 to 20 carbon atoms.

**30.** Catalyst, characterized in that it is composed, at least in part, of a material capable of being obtained by subjecting tungsten carbide to a hydrogenation treatment advantageously in the liquid phase, at a temperature at least equal to 50°C, advantageously to 100°C, at a pressure at least equal to 2 atmospheres, advantageously to 5 atmospheres, preferably to 10 atmospheres.

**31.** Catalyst according to claim 30, characterized in that the specific surface of the said material is between 1/10th and 1000 $m^2/g$, generally from 1 to 500 $m^2/g$.

**Patentansprüche**

**1.** Verfahren zur Hydrierung eines Nitro- oder Nitrosoderivats zu einer Amingruppe, wobei das Nitro- oder Nitrosoderivat in Gegenwart von Wolframcarbid hydriert wird, dadurch gekennzeichnet, daß die Hydrierung in flüssiger Phase erfolgt, der Partialdruck des Wasserstoffs mindestens fünf Atmosphären ($5 \cdot 10^5$ Pascal) und höchstens zweihundert Atmosphären ($2 \cdot 10^7$ Pascal) entspricht und das Nitro- oder Nitrosoderivat aromatisch ist und das Stickstoffderivat der folgenden Formel (I) entspricht:

$$(Z)_q(Y)_p(X)_n - Ar - (NO_m)_x$$

in der:

- Ar einen aromatischen Rest darstellt, der gegebenenfalls mit einer Alkylgruppe, die vorzugsweise 1 bis 4 Kohlenstoffatome enthält, eine Aralkylgruppe, eine Alkenylgruppe oder einer funktionellen Gruppe substituiert ist, wie z.B. einer Hydroxylgruppe, einer Trifluormethylgruppe, einer Nitrilgruppe, einer Säuregruppe, einer Estergruppe, einer Ketogruppe, einer $\alpha$-ungesättigten Säuregruppe, einer Ethergruppe oder einem Heterozyklus;
- X, Y und Z ein Halogen darstellen, das vorzugsweise ausgewählt ist aus Fluor, Chlor und Brom;
- x = 1, 2 oder sogar 3 ist;
- n, p und q eine ganze Zahl zwischen 0 und 5 darstellen;
  wobei die Summe n+p+q größer oder gleich 0 sein kann und m ausgewählt ist zwischen 1 und 2 und wobei die Verbindung höchstens 50 Kohlenstoffatome aufweist.

2. Verfahren nach Anspruch 1, dadurch gekennzeichnet, daß das Wolframcarbid dadurch aktiviert wird, daß man es einer Temperatur von mindestens 50°C, vorzugsweise 100°C, und einem Wasserstoffdruck von mindestens 2 Atmosphären, vorzugsweise 5 Atmosphären, unterwirft.

3. Verfahren nach Anspruch 1, dadurch gekennzeichnet, daß das Wolframcarbid dadurch aktiviert wird, daß man es den folgenden Bedingungen unterwirft:

- Temperaturen von mehr als oder gleich 150°C bei einem Wasserstoffpartialdruck von mindestens 2 Atmosphären;
- Drucken oberhalb von 10 Atmosphären, vorzugsweise 20 Atmosphären;
- einer Temperatur von mindestens 100°C und einem Druck oberhalb von 10 Atmosphären.

4. Verfahren nach Anspruch 3, dadurch gekennzeichnet, daß das Wolframcarbid in Gegenwart eines sauren wäßrigen Milieus aktiviert wird.

5. Verfahren nach Anspruch 4, dadurch gekennzeichnet, daß die Gruppe Ar einen monocyclischen aromatischen Rest darstellt und X und Y Chlor und/oder Fluor darstellen und die Summe aus n+p größer oder gleich 1 und kleiner oder gleich 3 ist.

6. Verfahren nach einem der Ansprüche 4 und 5, dadurch gekennzeichnet, daß q gleich Null ist.

7. Verfahren nach einem der Ansprüche 1 bis 6, dadurch gekennzeichnet, daß die Menge an zu verwendendem Katalysator zwischen 0,01 Gew.-% und 50 Gew.-% variiert, bezogen auf die Menge an eingesetztem Substrat.

8. Verfahren nach einem der Ansprüche 1 bis 7, dadurch gekennzeichnet, daß die Reaktion in Gegenwart einer starken Säure unter den Herstellungsbedingungen der Bamberger-Umlagerung durchgeführt wird.

9. Verfahren nach Anspruch 8, dadurch gekennzeichnet, daß die starke Säure ausgewählt ist aus Phophorsäuren, Schwefelsäure, Halogenwasserstoffsäuren und deren Mischungen.

10. Verfahren nach den Ansprüchen 8 und 9, dadurch gekennzeichnet, daß die starke Säure ausgewählt ist aus Chlorwasserstoffsäure oder Bromwasserstoffsäure.

11. Verfahren nach einem der Ansprüche 1 bis 10, dadurch gekennzeichnet, daß die Reaktion in Abwesenheit eines Lösungsmittels oder in einem Lösungsmittel durchgeführt wird, das ausgewählt ist aus Alkoholen, aromatischen Derivaten und deren Mischungen, wobei das Lösungsmittel vorzugsweise ausgewählt ist aus protischen Lösungsmitteln, insbesondere aus Wasser und Alkoholen, vorzugsweise Methanol und Mischungen, die es enthalten.

12. Verfahren nach einem der Ansprüche 1 bis 11, dadurch gekennzeichnet, daß die Reaktion bei einer Temperatur zwischen Umgebungstemperatur und der Siedetemperatur der Reaktionsmischung unter den Verfahrensbedingungen durchgeführt wird, vorzugsweise bei Temperaturen zwischen 20°C und 200°C, insbesondere bei Temperaturen zwischen 50°C und 150°C.

13. Verfahren nach einem der Ansprüche 1 bis 12, dadurch gekennzeichnet, daß die Reaktion unter einem Wasserstoffpartialdruck von 5 bis 100 bar, vorzugsweise von 5 bis 20 bar, durchgeführt wird.

14. Verfahren nach einem der Ansprüche 4 bis 13, dadurch gekennzeichnet, daß die Reaktion in Gegenwart einer Säure, die Anilide bilden kann, und bei einer Temperatur oberhalb von 100°C durchgeführt wird, vorzugsweise, wenn die Säure eine Carbonsäure ist, bei einer Temperatur von mindestens etwa 150°C.

15. Verfahren nach einem der Ansprüche 1 bis 14, dadurch gekennzeichnet, daß die Gruppe Ar $(Ed)_r$-Ar' ist, wobei Ed eine oder mehrere Gruppen, gleich oder verschieden, darstellt, die ausgewählt sind aus Alkylresten mit vorzugsweise 1 bis 10 Kohlenstoffatomen, Aralkylresten, Alkenylresten oder einer funktionellen Gruppe, wie z.B. einer Hydroxylgruppe, einer Trifluormethylgruppe, einer Nitrilgruppe, einer Säuregruppe, einer Estergruppe, einer Ketogruppe, einer $\alpha$-ungesättigten Säuregruppe, einer Ethergruppe, einem Heterozyklus, und wobei r eine ganze Zahl zwischen 0 und 3, vorzugsweise von 0, 1 oder 2, ist.

16. Verfahren nach Anspruch 15, dadurch gekennzeichnet, daß das Stickstoffderivat der Formel (II) entspricht

$$(Ed)_r\text{-}Ar' - (NO_m)_x \qquad (II)$$

wobei

Ed ein oder mehrere Gruppen, gleich oder verschieden, darstellt, die ausgewählt sind aus Alkylgruppen mit 1 bis 10 Kohlenstoffatomen, Aralkylgruppen, Alkenylgruppen oder einer funktionellen Gruppe, wie z.B. einer Hydroxylgruppe, einer Trifluormethylgruppe, einer Nitrilgruppe, einer Säuregruppe, einer Estergruppe, einer Ketogruppe, einer $\alpha$-ungesättigten Säuregruppe, einer Ethergruppe und einem Heterozyklus;
wobei
r eine ganze Zahl zwischen 0 und 3 ist, vorzugsweise ausgewählt aus 0, 1 oder 2;
wobei
m eine ganze Zahl zwischen 1 und 2 ist;
wobei
Ar' einen aromatischen Rest darstellt, der gegebenenfalls mit einer Alkylgruppe mit vorzugsweise 1 bis 4 Kohlenstoffatomen, einer Aralkylgruppe, einer Alkenylgruppe oder einer funktionellen Gruppe substituiert ist, wie z.B. Hydroxyl, Trifluormethyl, Nitril, Säurefunktion, Estergruppe, Ketogruppe, $\alpha$-ungesättigte Säuregruppe, Ethergruppe, Heterozyklus;
wobei
x = 1, 2 oder sogar 3 ist.

**17.** Verfahren nach einem der Ansprüche 4 bis 16, dadurch gekennzeichnet, daß die Anzahl an Ringen in dem Rest Ar höchstens 5, vorzugsweise 3, beträgt.

**18.** Verfahren nach einem der Ansprüche 16 und 17, dadurch gekennzeichnet, daß die Anzahl an Ringen im Rest Ar' höchstens 5, vorzugsweise 3, beträgt.

**19.** Verfahren nach einem der Ansprüche 16 bis 18, dadurch gekennzeichnet, daß die Reste Ed Alkoxyl-, Acyloxyl- und/oder Hydroxylreste sind.

**20.** Verfahren nach einem der Ansprüche 1 bis 19, dadurch gekennzeichnet, daß das Stickstoffderivat höchstens 50 Kohlenstoffatome, vorzugsweise höchstens 30 Kohlenstoffatome, aufweist.

**21.** Hydrierungsreagenz auf Basis vom Wolframcarbid, dadurch gekennzeichnet, daß es die folgenden Spezies enthält:

- ein Material, das erhalten werden kann, indem man Wolframcarbid einer Hydrierungsbehandlung, vorzugsweise in flüssiger Phase, bei einer Temperatur von mindestens 50°C, vorzugsweise 100°C, bei einem Druck von mindestens 5 Atmosphären, vorzugsweise 10 Atmosphären, unterwirft;
- eine flüssige Phase;
- Wasserstoff mit einem Druck von mindestens 2 Atmosphären, vorzugsweise 5 Atmosphären, insbesondere 10 Atmosphären, und höchstens zweihundert Atmosphären ($2 \cdot 10^7$ Pascal).

**22.** Reagenz nach Anspruch 21, dadurch gekennzeichnet, daß die Flüssigphase ein Lösungsmittel umfaßt, das ausgewählt ist aus Alkoholen, aromatischen Derivaten und deren Mischungen, wobei das Lösungsmittel vorzugsweise ausgewählt ist aus protischen Lösungsmitteln, insbesondere aus Wasser und Alkoholen, vorzugsweise aus Methanol und Mischungen, in denen es enthalten ist.

**23.** Reagenz nach den Ansprüchen 21 und 22, dadurch gekennzeichnet, daß die flüssige Phase einen Säuregehalt von mindestens 0,1 N, vorzugsweise von 1 N, aufweist.

**24.** Reagenz nach den Ansprüchen 21 bis 23, dadurch gekennzeichnet daß die flüssige Phase eine starke Säure umfaßt, die ausgewählt ist aus Schwefelsäuren, Sulfonsäuren, Phosphorsäuren, Carbonsäuren, Halogenwasserstoffsäuren und deren Mischungen.

**25.** Reagenz nach den Ansprüchen 21 bis 24, dadurch gekennzeichnet, daß die flüssige Phase eine starke Säure umfaßt, die bei der Bamberger-Umlagerung verwendet werden kann.

**26.** Reagenz nach Anspruch 25, dadurch gekennzeichnet, daß die flüssige Phase eine starke Säure umfaßt, die ausgewählt ist aus Phosphorsäuren, Schwefelsäure, Halogenwasserstoffsäuren und deren Mischungen.

**27.** Reagenz nach den Ansprüchen 25 bis 26, dadurch gekennzeichnet, daß die flüssige Phase eine starke Säure umfaßt, die ausgewählt ist aus Chlorwasserstoffsäure oder Bromwasserstoffsäure.

**28.** Reagenz nach den Ansprüchen 21 bis 27, dadurch gekennzeichnet, daß die flüssige Phase eine Carbonsäure umfaßt.

**29.** Reagenz nach Anspruch 28, dadurch gekennzeichnet, daß die Carbonsäure eine Monosäure mit 1 bis 20 Kohlenstoffatomen ist.

**30.** Katalysator, dadurch gekennzeichnet, daß er mindestens zum Teil aus einem Material besteht, das erhalten werden kann, indem man Wolframcarbid einer Hydrierungsbehandlung, vorzugsweise in flüssiger Phase, bei einer Temperatur von mindestens 50°C, vorzugsweise 100°C, bei einem Druck von mindestens 2 Atmosphären, vorzugsweise 5 Atmosphären, insbesondere 10 Atmosphären, unterwirft.

**31.** Katalysator nach Anspruch 30, dadurch gekennzeichnet, daß die spezifische Oberfläche des Materials zwischen 1 $m^2/g$ und 1000 $m^2/g$, im allgemeinen zwischen 1 und 500 $m^2/g$, liegt.